Europäisches Patentamt

**European Patent Office**

**Office européen des brevets**

(11) Numéro de publication : **0 042 783 B1**

# FASCICULE DE BREVET EUROPÉEN

(45) Date de publication du fascicule du brevet :
**21.03.84**

(51) Int. Cl.³ : **A 61 F   1/00// C23F7/08**

(21) Numéro de dépôt : **81400939.5**

(22) Date de dépôt : **15.06.81**

(54) **Procédé pour la réalisation de revêtements bioactifs sur des prothèses osseuses, et prothèses ainsi obtenues.**

(30) Priorité : **17.06.80 FR 8013433**

(43) Date de publication de la demande :
**30.12.81 Bulletin 81/52**

(45) Mention de la délivrance du brevet :
**21.03.84 Bulletin 84/12**

(84) Etats contractants désignés :
**BE CH DE GB IT LI NL**

(56) Documents cités :
**FR-A- 2 027 413**
**FR-A- 2 336 913**
**FR-A- 2 374 019**
**GB-A-   863 098**

(73) Titulaire : **SOCIETE EUROPEENNE DE PROPULSION**
**Société Anonyme dite**
**3, avenue du Général de Gaulle**
**F-92800 Puteaux (FR)**

(72) Inventeur : **Ghommidh, Josette**
**Résidence Les Ormes -BT·A 2**
**F-31 320 Castanet Tolozan (FR)**
Inventeur : **Buttazzoni, Bernard**
**La Gallon 23 Bd A. Cieussa**
**F-13007 Marseille (FR)**
Inventeur : **Constant, Georges**
**1 Rond-Point de la Paburbière**
**F-31520 Ramouville St Agne (FR)**
Inventeur : **Diloy, Etienne**
**16 rue de la Gironde**
**F-31300 Toulouse (FR)**
Inventeur : **Morancho, Roland**
**69-71 rue P. Cazeneuve**
**F-31200 Toulouse (FR)**

(74) Mandataire : **Combe, André et al**
**CABINET BEAU DE LOMENIE 55 rue d'Amsterdam**
**F-75008 Paris (FR)**

Procédé pour la réalisation de revêtements bioactifs sur des prothèses osseuses, et prothèses ainsi obtenues

La présente invention concerne un procédé pour la réalisation de revêtements bioactifs sur des prothèses osseuses et les prothèses ainsi obtenues.

Les prothèses osseuses (telles que les prothèses articulaires et les implants dentaires) utilisées actuellement comportent une partie destinée à être fixée à demeure dans l'os compact ou spongieux ou dans une cavité de l'os. Pour maintenir en place ces prothèses, on utilise soit un ciment interposé entre la prothèse et l'os, soit un ancrage mécanique par emmanchement à force de la prothèse dans une cavité dont la paroi a été rendue rugueuse ; dans ce dernier cas, le développement de l'os, après la mise en place de la prothèse, provoque un renforcement de l'ancrage de la prothèse sans qu'il y ait une soudure entre l'os et la prothèse.

Par ailleurs, on sait que certains solides minéraux participent aux mécanismes physiologiques des êtres vivants, ainsi les phosphates de calcium, plus particulièrement les apatites, sont des composés solides qui se forment au niveau des tissus calcifiés (os et dents) des vertébrés. Ces composés biologiques remplissent deux fonctions, l'une mécanique (résistance aux efforts), l'autre physiologique (métabolisme du phosphore et du calcium). On a récemment découvert certaines relations se manifestant à l'interface entre un implant dont la surface est pourvue de phosphate (ou phosphates) de calcium et l'os voisin sous forme d'une véritable soudure biochimique entre l'os et l'implant. Ainsi, on sait que le phosphate tricalcique β peut être « assimilé » par le tissu osseux et remplacé par ledit tissu, on sait également que l'hydroxyapatite de calcium (notamment s'il présente une certaine déficience en calcium) présente une structure cristalline très proche de la structure osseuse et peut de ce fait servir de structure réceptrice vis-à-vis d'un développement de l'os ; on sait enfin que, vis-à-vis d'une structure osseuse voisine, le pyrophosphate γ ou β (principalement le pyrophosphate γ) joue un rôle important dans la liaison (adhésion) entre la structure osseuse et l'implant. Il est donc important de disposer d'une technique permettant le dépôt, sur la surface d'une base d'implant en matériau inerte, d'un revêtement bioactif à base de phosphate de calcium ou d'un mélange de phosphates de calcium convenablement choisis. C'est le but de la présente invention.

Ainsi, selon la présente invention, on réalise un revêtement bioactif mince, de phosphate (ou phosphates) de calcium, sur un implant en un matériau inerte de façon à favoriser la soudure biochimique entre l'os et l'implant ainsi revêtu.

Le matériau inerte utilisé pour réaliser la base de l'implant peut être un alliage métallique (acier inoxydable qualité chirurgicale), un alliage cobalt-chrome-nickel-molybdène, un titane allié, un polymère, une céramique, un matériau carboné (fibres de carbone), etc. Ces derniers matériaux (fibres de carbone-matrice pyrocarbone notamment) sont particulièrement intéressants grâce, d'une part, à la biocompatibilité intrinsèque du carbone vis-à-vis du tissu osseux et, d'autre part, à la bioflexibilité de ces matériaux, cette bioflexibilité étant la propriété desdits matériaux d'avoir un module d'élasticité proche de celui de l'os.

Ainsi, selon l'invention, on réalise sur un implant en matériau inerte un revêtement bioactif mince à base d'un phosphate de calcium. Parmi les phosphates de calcium utilisables selon l'invention, on citera les apatites, les hydroxyapatites, le phosphate tricalcique β, le pyrophosphate de calcium γ et β, la brushite ou la monétite. Ces phosphates de calcium sont utilisés seuls ou en mélange entre eux, sous une forme amorphe, partiellement cristalline ou cristalline et ils peuvent comporter d'autres matériaux (par exemple des ions métalliques ou d'autres composés minéraux) qui seront susceptibles d'améliorer leurs propriétés (par exemple l'adhésivité sur le matériau inerte ou les propriétés de surface).

Le revêtement bioactif doit être mince, c'est-à-dire qu'il doit avoir une épaisseur comprise entre 2 et 15 μm, de façon à jouer, vis-à-vis de l'os en contact duquel il est placé, un rôle aussi bénéfique que possible (accrochage et assimilation).

Le procédé selon l'invention pour réaliser le revêtement bioactif mince, à base de phosphate de calcium, sur l'implant inerte est caractérisé en ce qu'on réalise, à partir de solutions aqueuses contenant un phosphate de calcium, tel qu'un phosphate bicalcique, un brouillard de particules d'eau contenant ledit phosphate, que l'on entraîne ledit brouillard à l'aide d'un courant gazeux convenable, et que l'on oriente ledit courant gazeux sur l'implant à revêtir alors que ledit courant et ledit implant sont chauffés à température convenable pour provoquer la transformation chimique dudit phosphate de calcium contenu dans la solution aqueuse de départ en au moins un phosphate de calcium bioactif.

La solution aqueuse de départ est de préférence une solution de phosphate bicalcique hydraté de formule $CaHPO_4$, $2 H_2O$. La solution aquese comportera de préférence une quantité aussi grande que possible de ce phosphate bicalcique hydraté, c'est-à-dire qu'elle sera voisine de la concentration de saturation (à température ordinaire) dudit phosphate bicalcique. (La concentration optimale sera ainsi de l'ordre de 120 mg de phosphate par litre de solution).

A partir de cette solution, on réalise un brouillard (c'est-à-dire le fractionnement de ladite solution en fines gouttelettes) par un dispositif quelconque. C'est ainsi que l'on peut utiliser un atomiseur pneumatique ou une cavité résonnante ou un transducteur piézo-électrique. Les gouttelettes dudit brouillard ont avantageusement, mais de façon non limitative, des diamètres de l'ordre de 5 à 50 ou 100 µm.

Les gouttelettes dudit brouillard sont entraînées par un gaz vecteur tel que l'azote, l'argon, l'air, de façon à être amenées au contact de l'implant à revêtir. L'expérience prouve qu'il est souhaitable de prendre des dispositions (chauffage) pour éviter la condensation de ces gouttelettes sur les parois des tubes dans lesquels elles sont entraînées et de prévoir une orientation spécifique du courant de gouttelettes vers ledit implant pour favoriser le contact réel entre lesdites gouttelettes (ou les produits desséchés qu'elles contiennent) avec la surface dudit implant.

Les gouttelettes entraînées par le courant de gaz porteur sont envoyées dans un four dont la température est comprise entre 100 et 350 °C, puis sur la surface de l'implant qui est lui-même à une température comprise entre 150 et 600 °C. Au cours du chauffage des gouttelettes, il se produit, d'une part, une vaporisation plus ou moins complète de l'eau de ces gouttelettes et, d'autre part, en phase liquide, une hydrolyse donnant naissance au moins partiellement au phosphate bioactif déposé : cette transformation du phosphate bicalcique se trouvant dans l'eau sous une forme plus ou moins ionique en un (ou plusieurs) phosphate bioactif déposé sur la surface du substrat se trouve réalisée, selon les conditions expérimentales, soit avant le dépôt du phosphate sur l'implant, soit, le plus souvent, au moment du contact des particules (gouttelettes ou particules solides) avec la surface du substrat. Cette réaction au moment du contact entre les particules et la surface rencontrée explique semble-t-il le fait que la nature du matériau qui se forme sur la surface paraît dépendre de la nature de cette surface.

Dans un des modes préférés de l'invention, le courant gazeux comportant les gouttelettes et l'implant sont chauffés dans un même four à une température comprise entre 150 et 350 °C ; de plus, la durée du trajet des gouttelettes dans le four est contrôlée de façon que l'évaporation de l'eau desdites gouttelettes soit totale avant l'arrivée de ces gouttelettes sur la surface de l'implant.

La pression à laquelle est·réalisé le rendement de l'implant est en général égale à la pression atmosphérique. Cependant, il peut être intéressant, notamment pour accélérer les transformations chimiques au sein des gouttelettes et pour augmenter le rendement, d'opérer sous une pression supérieure à la pression atmosphérique.

Comme il a été indiqué ci-dessus, le procédé se prête parfaitement à la réalisation de couches d'hydroxyapatite, de phosphate tricalcique β, de pyrophosphate de calcium γ et β, de brushite et de monétite, seuls ou en mélange, suivant les conditions opératoires, mais aussi à la réalisation de couches multiples en modifiant les paramètres opératoires en cours d'élaboration ; grâce à la possibilité de choisir les conditions opératoires, on pourra, compte tenu de la nature de l'implant et des nécessités d'utilisations ultérieures, réaliser des dépôts contenant des proportions déterminées de tel ou tel phosphate bioactif et/ou inerte. On a notamment pu réaliser des dépôts formés (ou contenant) de l'hydroxyapatite déficient en calcium qui, d'après les connaissances actuelles, constitue le matériau dont la structure se rapproche le plus de la structure de l'os.

Le substrat, compte tenu de la température de dépôt, peut être choisi parmi un très grand nombre de matériaux : aciers inoxydables, aciers spéciaux réfractaires, titane pur ou allié, matériaux composites carbone-carbone.

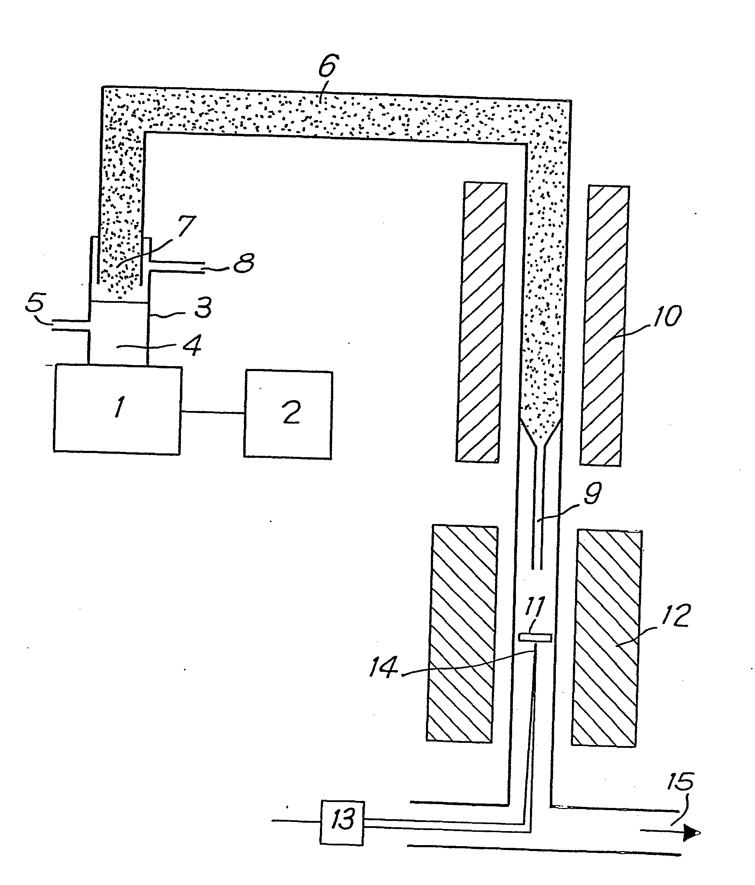

Le procédé de l'invention ayant été exposé dans sa forme générale, la description qui suit fournit plusieurs exemples de mise en œuvre destinés à illustrer celui-ci ; ces exemples ont été conduits au moyen d'un appareillage du type de celui qui est schématisé à la figure unique et qui est décrit préalablement ci-après en référence à cette figure.

Cet appareillage comprend essentiellement :

— un générateur d'ultrasons 1 dont l'alimentation électrique est figurée en 2 ;

— un récipient 3 contenant le liquide à pulvériser 4, flanqué d'une tubulure d'admission de liquide 5 ;

— une conduite d'amenée 6 du brouillard 7 portant au départ la tubulure d'admission du gaz vecteur 8 et à la sortie d'une buse 9 qui se trouve, par exemple, à une distance de 10 à 70 mm du substrat (implant) 11 ; dans la partie finale, cette conduite est préchauffée par le système 10 ;

— le substrat (implant) 11, chauffé par le four 12 à la température fixée par un système de régulation 13 et son thermocouple 14 ;

— une évacuation 15 du brouillard n'ayant pas réagi, du gaz vecteur et des produits de décomposition.

Le mode opératoire pour la réalisation d'un dépôt comprend les étapes suivantes :

— préparation de la solution à pulvériser et mise en place dans le récipient 3 ;

— introduction du substrat 11 dans le four 12 ;

— mise en balayage du conduit 6 par le gaz vecteur et réglage de son débit ;

— mise en température du four 12 ;

— mise en route du système de préchauffage 10 ;

— mise en route du générateur d'ultrasons 1 et réglage de la puissance utilisée ;

— à la fin de l'expérimentation, on arrête successivement le générateur d'ultrasons, le chauffage du four 12, du dispositif de préchauffage 10 ; la circulation du gaz vecteur est arrêtée lorsque la · température du four est descendue en-dessous de 80 °C.

Bien entendu, cet appareillage et ce mode opératoire sont donnés uniquement à titre d'illustration.

Plusieurs exemples de dépôt réalisés sont indiqués ci-dessous.

Exemple 1

Dépôt d'hydroxyapatite calcique

La solution aqueuse de départ est réalisée en dissolvant 120 mg de phosphate bicalcique hydraté ($CaHPO_4$, $2 H_2O$) dans 1 litre d'eau distillée. La pulvérisation chimique est mise en œuvre durant 4 h avec un débit de 6 litres/minute d'azote, le substrat constitué d'une plaquette de matériau composite carbone-carbone étant chauffé à 350 °C, la température du préchauffage étant, elle, de 300 °C.

On observe en fin d'expérience une couche cristalline blanche d'hydroxyapatite calcique sur le substrat. La nature de cette couche a été identifiée par diffraction de rayons X et par observation des émissions secondaires sous un faisceau d'électrons avec un appareillage de type « E.D.A.X. » L'épaisseur de cette couche mesurée par microscopie électronique à balayage est en cet exemple d'environ 4 μm.

Exemple 2

Dépôt d'hydroxyapatite calcique
Les conditions de cette expérimentation sont les· mêmes qu'en 1, à l'exception du gaz vecteur qui est constitué par de l'argon dans les mêmes conditions de débit. Les résultats sont identiques.

Exemple 3

Dépôt d'hydroxyapatite calcique
Les conditions de cette expérimentation sont les mêmes qu'en 1, à l'exception de la solution de départ préparée à partir d'hydroxyde de calcium et d'acide phosphorique, les quantités relatives d'acide phosphorique et d'hydroxyde correspondant sensiblement au phosphate tricalcique. On réalise ainsi un dépôt d'hydroxyapatite calcique.

Exemple 4

Dépôt d'hydroxyapatite calcique
Les conditions de cette expérimentation sont les mêmes qu'en 1, à l'exception :
— de la température du substrat qui est de 300 °C,
— du débit du gaz vecteur qui est de 8 litres/minute.
La durée totale du dépôt a été de 8 heures.
La couche déposée est de l'hydroxyapatite de calcium ; elle a une épaisseur d'environ 7 μm.
Les dépôts d'hydroxyapatite sont des produits de formule $Ca_{10}(PO_4)_6(OH)_2$.

Exemples 5 et 6

Dans les mêmes conditions que celles décrites dans les exemples 1 à 4, mais en faisant varier le débit de la solution de départ, on peut obtenir des dépôts contenant, outre l'hydroxyapatite, soit du phosphate bicalcique anhydre, soit du γ-pyrophosphate, soit un mélange de ces deux produits.
L'augmentation de la température du support (vers 400 °C) permet également l'obtention, dans le dépôt, d'une phase de γ-pyrophosphate.
Enfin, il a été remarqué que la prolongation de la durée des dépôts permet, toutes choses égales par ailleurs, de modifier la nature du dépôt (par exemple par formation de pyrophosphate, puis d'hydroxyapatite) non pas du fait d'une transformation chimique des dépôts déjà réalisés, mais du fait que les dépôts s'effectuent sur des surfaces dont la nature varie au cours de la durée de l'essai.

Exemple 7

Dépôt de pyrophosphate de calcium forme γ
Les conditions de cette expérimentation sont les mêmes qu'en 4, à l'exception :
— de la température du substrat qui est de 400 ou 500 °C,
— de la durée de circulation du brouillard sur le substrat qui est de 3 heures.
La couche déposée est constituée de pyrophosphate de calcium forme γ d'épaisseur d'environ 3 μm.

Exemple 8

Dépôt de pyrophosphate de calcium forme β
Les conditions de cette expérimentation sont les mêmes qu'en 7, à l'exception de la température du substrat qui est de 600 °C.
La couche déposée est constituée de pyrophosphate de calcium forme β d'épaisseur d'environ 3 μm.
La température influe donc fortement sur la nature du dépôt.

Exemple 9

Dépôt de monétite
Les conditions de cette expérimentation sont les mêmes qu'en 1, à l'exception :
— de la température du substrat qui est de 400 °C,
— du débit du gaz vecteur qui est de 5 litres/minute,
— de la température du système de préchauffage qui est de 100 °C.
La couche déposée est constituée de monétite $CaHPO_4$ d'épaisseur d'environ 2,5 μm.
Cette expérimentation montre l'influence du préchauffage.

Exemple 10

Dépôt d'hydroxyapatite calcique
Les conditions de cette expérimentation sont les mêmes qu'en 1, à l'exception :
— du substrat qui est une plaque de polytétrafluoréthylène,
— de la température du substrat qui est de 250 °C,
— de la température de préchauffage qui est de 200 °C.
La couche déposée d'environ 4 μm est constituée d'hydroxyapatite calcique et de traces de γ-pyrophosphate de calcium.

Exemple 11

Dépôt de monétite

Les conditions de cette expérimentation sont les mêmes qu'en 10, à l'exception du substrat qui est en alumine frittée.

La couche déposée est de la monétite, montrant que les dépôts peuvent aussi être réalisés sur les céramiques, toutefois avec une influence du substrat sur la nature du dépôt.

Exemple 12

Dépôt de monétite

Les conditions de cette expérimentation sont les mêmes qu'en 10, à l'exception du substrat qui est en acier inoxydable 18/8.

La couche déposée est de la monétite montrant que les métaux peuvent aussi être utilisés comme substrat.

Exemple 13

Dépôt d'hydroxyapatite calcique et de γ-pyrophosphate de calcium en mélange

Les conditions de cette expérimentation sont les mêmes qu'en 1, à l'exception :

— de la composition de la solution aqueuse de départ qui est obtenue en dissolvant 180 mg de phosphate monocalcique hydraté $CaH_4(PO_4)_2$, $H_2O$ dans un litre d'eau distillée,

— de la température du substrat qui est de 300 °C,

— de la température de préchauffage qui est de 280 °C,

— du débit du gaz vecteur qui est de 5 litres/minute.

La couche déposée est un mélange d'hydroxyapatite calcique et de γ-pyrophosphate de calcium, ce dernier composé étant prépondérant.

**Revendications**

1. Procédé pour la réalisation de revêtements bioactifs sur des prothèses osseuses, caractérisé en ce que :

— on réalise, par des moyens connus, un brouillard d'une solution concentrée d'un phosphate de calcium, de préférence de phosphate bicalcique,

— on entraîne ledit brouillard à l'aide d'un courant gazeux,

— on porte ledit brouillard à une température comprise entre 100 et 350 °C en l'envoyant sur le support de la future prothèse osseuse, ledit support étant lui-même maintenu à une température comprise entre 200 et 600 °C, et on provoque ainsi une transformation chimique du phosphate calcique présent dans la solution de départ en au moins un phosphate bioactif déterminé parmi l'hydroxyapatite, le phosphate tricalcique β, le pyrophosphate de calcium γ et β, le brushite et la monétite ou des mélanges de ces phosphates,

— les conditions opératoires étant choisies de façon que l'épaisseur dudit revêtement soit comprise entre 2 et 15 μm.

2. Implants osseux à surface bioactive, caractérisés en ce qu'ils comportent un revêtement obtenu selon le procédé de la revendication 1.

**Claims**

1. Process for making bioactive coatings on osseous prostheses, characterized in that :

— a mist of a concentrated solution of a phosphate of calcium, preferably dicalcium phosphate is made by known means,

— said mist is entrained with the aid of a gaseous current,

— said mist is taken to a temperature of between 100 and 350 °C by sending it onto the support of the future osseous prosthesis, said support itself being maintained at a temperature of between 200 and 600 °C, and a chemical transformation is thus provoked of the calcium phosphate present in the starting solution into at least one bioactive phosphate determined from hydroxyapatite, β form tricalcium phosphate, γ and β form calcium pyrophosphate, brushite and monetite or mixtures of these phosphates,

— the operational conditions being chosen so that the thickness of said coating is between 2 and 15 μm.

2. Osseous implants with bioactive surface, characterized in that they comprise a coating obtained in accordance with the process of claim 1.

**Ansprüche**

1. Verfahren zur Herstellung von bioaktiven Überzügen für Knochenprothesen, dadurch gekennzeichnet, daß :

— mit bekannten Mitteln ein Nebel aus einer konzentrierten Lösung eines Calciumphosphats, vorzugsweise Dicalciumphosphat, hergestellt wird,

— der Nebel mittels eines Gasstroms mitgerissen wird,

— der Nebel unter Aufbringen auf die Unterlage der künftigen Knochenprothese auf eine Temperatur zwischen 100 und 350 °C gebracht wird, wobei die Unterlage selbst auf einer Temperatur zwischen 200 und 600 °C gehalten wird, und so eine chemische Umwandlung des in der Ausgangslösung vorhandenen Calciumphosphats in zumindest ein bioaktives Phosphat, wie Hydroxylapatit, β-Tricalciumphosphat, γ- und β-Calciumpyrophosphat, Brushit und Monetit oder Mischungen dieser Phosphate, bewirkt wird,

— die Arbeitsbedingungen derart gewählt werden, daß die Dicke des Überzugs zwischen 2 und 15 μm liegt.

2. Knochenimplantate mit bioaktiver Oberfläche, dadurch gekennzeichnet, daß sie einen nach dem Verfahren des Anspruchs 1 erhaltenen Überzug aufweisen.